# EUROPEAN PATENT SPECIFICATION

(11) **EP 3 251 508 B1**
(45) Date of publication and mention of the grant of the patent: **15.04.2020**
(21) Application number: 17161850.7
(22) Date of filing: 23.04.2010
(51) Int. Cl.: A01N 25/30, C07C 43/10, C07C 43/11, C07C 43/15, C11D 1/722, A01N 43/653, A01N 43/90, A01N 41/06, A01N 41/10, A01N 47/36, A01N 45/02, A01N 51/00, A01P 3/00, A01P 13/00, A01P 7/04

(54) **ALCOHOL ALKOXYLATES AS ADJUVANTS FOR AGROCHEMICAL FORMULATIONS**
ALKOHOLALKOXYLATE ALS ADJUVANZIEN FÜR AGROCHEMISCHE FORMULIERUNGEN
ALCOXYLATES D'ALCOOL COMME ADJUVANTS DANS DES FORMULATIONS AGROCHIMIQUES

(30) Priority: 23.04.2009 GB 0907003
(43) Date of publication of application: 06.12.2017
(62) Divisional of application: 10718253.7
(73) Proprietor: Syngenta Limited, Bracknell, Berkshire RG42 6EY (GB)
(72) Inventor: BELL, Gordon, Alastair, Bracknell, Berkshire RG42 6EY (GB)
(74) Representative: SYNGENTA IP

(56) References cited:
- EP-A2- 0 356 812
- WO-A1-2008/132150
- WO-A2-2005/084435
- US-A- 3 843 706
- DATABASE WPI Week 200560 Thomson Scientific, London, GB; AN 2005-585477 XP002592674, & JP 2005 200640 A (NIPPON OILS & FATS CO LTD) 28 July 2005 (2005-07-28)

## Description

This invention relates to bioperformance enhancing adjuvants and to use of such adjuvants.

Numerous adjuvants which enhance the bioperformance of agrochemicals are known. A broad mention of alcohol alkoxylates as penetration aids is made in WO2008/037375 and in WO2008132150.

The present invention provides a bioperformance enhancing adjuvant of formula (I):

R₁O[BO]ₙ[AO]ₘR₂ (I)

where BO is butylene oxide; R₂ is H and R₁ is C₁₂₋₁₅ alkyl or oleyl, m=0 and n=4.

BO [butylene oxide] has the empirical formula C₄H₈O. Every BO unit has the formula CH(R₄)CH(R₅)O but each BO unit is independently selected from the following options: R₄ is methyl and R₅ is methyl; or R₄ is ethyl and R₅ is hydrogen; or R₄ is hydrogen and R₅ is ethyl.

For m=0. Adjuvants for which m=0 may display very low levels of phytotoxicity [i.e. little damage to plants].

The bioperformance enhancing adjuvants of the present invention may be used effectively at much lower concentrations than the effective concentrations for conventional adjuvants.

The bioperformance enhancing adjuvants of the present invention may be used synergistically with other bioperformance enhancing adjuvants of the present invention or with conventional adjuvants.

The values of n and m represent values both for individual species and for averages taken over a distribution of compounds. This will be well understood by the skilled person.

Suitably the bioperformance enhancing adjuvants of the present invention are used to enhance the bioperformance of a pesticide. Pesticides suitable for use with the present invention include insecticides, fungicides, herbicides, acaricides, nematicides and biocides suitable for controlling pests, diseases or weeds that are a problem in agriculture. Many such pesticides are known and are described in The Pesticide Manual 14th edition published by the British Crop Protection Council in 2006.

The invention is illustrated by examples 26 to 32. All parts and percentages are by weight unless otherwise stated.

### EXAMPLE 1

Entry 28 and 29 illustrate compounds of the present invention. Twenty eight butylene oxide based compounds were synthesised using standard techniques familiar to a skilled person [for example, see EP0681865A]. Each sample consists of a hydrocarbon tail connected to a butylene oxide section which in turn is connected to an ethylene oxide section; the samples are compounds of formula (I) in which AO is ethylene oxide and R₂ is hydrogen; and R₁, n and m are as defined in Table 1. Samples 1 to 3 and 24 to 27 were prepared using branched alcohols containing an average of 13 carbon atoms. Samples 4 to 7 were prepared with 2-ethylhexyl alcohol. Samples 8 to 11 were prepared using butanol as the starting solvent. Samples 12 to 14 and sample 28 used a fraction of alcohols ranging from 12 to 15 carbons in length. Samples 15 to 23 used an alcohol range from C₁₂ to C₁₆. The values of n and m are average values.

**Table 1**

| **Sample** | **R₁ alkyl chain** | **n** | **m** |
|---|---|---|---|
| 1 | C₁₃ [average] | 2 | 8 |
| 2 | C₁₃ [average] | 4 | 8 |
| 3 | C₁₃ [average] | 6 | 12 |
| 4 | 2-ethylhexyl | 2 | 6 |
| 5 | 2-ethylhexyl | 2 | 3 |
| 6 | 2-ethylhexyl | 4 | 6 |
| 7 | 2-ethylhexyl | 6 | 10 |
| 8 | C₄ | 2 | 8 |
| 9 | C₄ | 4 | 10 |
| 10 | C₄ | 4 | 8 |
| 11 | C₄ | 6 | 8 |
| 12 | C₁₂₋₁₅ | 2 | 9 |
| 13 | C₁₂₋₁₅ | 4 | 10 |
| 14 | C₁₂₋₁₅ | 6 | 12 |
| 15 | C₁₂₋₁₆ | 1 | 3 |
| 16 | C₁₂₋₁₆ | 1 | 5 |
| 17 | C₁₂₋₁₆ | 1 | 8 |
| 18 | C₁₂₋₁₆ | 2 | 5 |
| 19 | C₁₂₋₁₆ | 2 | 8 |
| 20 | C₁₂₋₁₆ | 3 | 5 |
| 21 | C₁₂₋₁₆ | 3 | 8 |
| 22 | C₁₂₋₁₆ | 4 | 3 |
| 23 | C₁₂₋₁₆ | 4 | 8 |
| 24 | C₁₃ [average] | 2 | 3 |
| 25 | C₁₃ [average] | 2 | 8 |
| 26 | C₁₃ [average] | 4 | 3 |
| 27 | C₁₃ [average] | 4 | 8 |
| 28 | C₁₂₋₁₅ | 4 | 0 |
| 29 | C₁₈ (oleyl) | 4 | 0 |
| 30 | C₁₈ (oleyl) | 4 | 20 |

### EXAMPLE 2

This example shows that samples 15, 17, 24 and 25 from Table 1 behave as adjuvants for fungicides used against brown rust (puccinia recondita). Wheat was grown outside in field plots. Each plot was sprayed with water at a rate of 15 litres per hectare, the water containing either difenoconazole or cyproconazole, at a concentration which enabled a pesticide application rate of 0.1, 1, 3, 10 or 30 grams per hectare. Adjuvants were added at the standard rate of 0.2% v/v of the spray volume used. The known adjuvants Brij™ 96 and TEHP (tris 2-ethylhexyl phosphate) were also tested for comparison. Each experiment was replicated four times and the results were averaged at each rate. Plants were examined for both protective action and curative effects. A standard mathematical analytical technique was used: Plots of efficacy against pesticide concentration for each adjuvant were 'logit' transformed and used to estimate the concentration required for 90% effect (ED90). For each sample, its ED90 value was compared to that of the known adjuvant Brij™96 or TEHP in order to generate a relative potency; the relative potency is the ratio of the ED90 values. Relative potencies are given in Table 2 [relative potency results compared to Brij™96 for difenoconazole] and Table 3 [relative potency results compared to TEHP for cyproconazole]. Adjuvants which performed better than the standard adjuvants have a relative potency value greater than 1.

**Table 2**

| **Sample** | **Protective** | **Curative** |
|---|---|---|
| 15 | 0.99 | 1.50 |
| 17 | 1.47 | 1.69 |
| 24 | 1.46 | 2.84 |
| 25 | 1.22 | 1.07 |

**Table 3**

| **Sample** | **Protective** | **Curative** |
|---|---|---|
| 15 | 1.80 | 3.81 |
| 17 | 0.97 | 2.73 |
| 24 | 1.68 | 2.97 |
| 25 | 1.22 | 4.29 |

### EXAMPLE 3

In this example Sample 13 from Table 1 was compared to the oil adjuvant blend Turbocharge™. The herbicide fomesafen was applied to the weed species xanthium strumarium (XANST), setaria viridis (SETVI), abutilon theophrasti (ABUTH), and chenopodium album (CHEAL) at rates of 60 or 120 grams per hectare, using a laboratory track sprayer. Each experiment was replicated four times and the percentage damage to the weeds was assessed visually at time periods of 7, 14 and 21 days after application. Each adjuvant was applied at a rate of 0.5% of the volume of the spray water. Four effects were evaluated: pesticide rate (2 levels), adjuvant type (two levels), weed species (four levels) and days after application (three levels). A standard simple linear model was constructed to judge the significance of these effects. These effects were found to be significant at a 5% level. A model was fitted using multiple linear regression using the statistics package JMP (SAS group). The effect of each adjuvant was pulled out from the model and the least significant differences evaluated using a Student's t method. In further examples, where a greater number than two adjuvants were compared, Tukey's HSD method was used.

Table 4 shows the mean efficacy of the butylene oxide adjuvant number 13 with the pesticide fomesafen compared to the oil adjuvant Turbocharge across four weed species and compared to single weed species. The mean values and a letter code denoting significant difference (samples with the same letter are not significantly different from each other at the 5% level.) In addition the mean values split according to individual weed species are also shown. As can be seen, Sample 13 was more efficacious than the standard adjuvant Turbocharge™ across the weed species, and was as good or better on individual weeds.

**Table 4**

| **Sample** | **All weeds** | **XANST** | **CHEAL** | **SETVI** | **ABUTH** |
|---|---|---|---|---|---|
| Sample 13 | 61.8; A | 89.7; A | 89.4; A | 27.5; A | 40.55; A |
| Turbocharge | 58.8; B | 83.9; B | 88.6; A | 24.4; B | 38.33; A |

### EXAMPLE 4

In this example Sample 13 from Table 1 was compared to the adjuvant Brij™96V. The herbicide fomesafen was applied to the weed species xanthium strumarium (XANST), setaria viridis (SETVI), abutilon theophrasti (ABUTH), and chenopodium album (CHEAL) at rates of 60 or 120 grams per hectare by a laboratory track sprayer. Each experiment was replicated four times and the percentage damage to the weeds was assessed visually at time periods of 7, 14 and 21 days after application. Each adjuvant was applied at a rate of 0.2% of the volume of the spray water.

The same statistical methodology that was applied in Example 3 was used here. Table 5 shows the mean efficacy of the butylene oxide adjuvant number 13 with the pesticide fomesafen compared to the adjuvant Brij 96 across four weed species and compared to single weed species. The mean values and a letter denoting which group each adjuvant belonged to are shown. In addition the mean values split according to individual weed species are also shown. As can be seen the butylene oxide adjuvant was more efficacious than the standard adjuvant across the weed species, and was as good or better on individual weeds.

**Table 5**

| **Sample** | **All weeds** | **ABUTH** | **CHEAL** | **SETVI** | **XANST** |
|---|---|---|---|---|---|
| Sample 13 | 57.15; A | 32.5; A | 85.27; A | 24.17; A | 86.66; A |
| Brij 96V | 53.33; B | 32.2; A | 82.5; A | 26.66; A | 71.94; B |

### EXAMPLE 5

In this example the rate response of the butylene oxide adjuvant number 13 of Table 1 was measured to display the excellent performance of this adjuvant at very low rates. compared to the adjuvant Brij™96V. The herbicide fomesafen was applied to the weed species xanthium strumarium (XANST), setaria viridis (SETVI), abutilon theophrasti (ABUTH), and chenopodium album (CHEAL) at rates of 60 or 120 grams per hectare by a laboratory track sprayer. Each experiment was replicated four times and the percentage damage to the weeds was assessed visually at time periods of 7, 14 and 21 days after application. The adjuvants were each applied at a rate of 0.2% of the volume of the spray water.

The same statistical methodology that was applied in Example 3 was used here. Where a sample has more than one letter it is not significantly different to any other sample with one of those letters.

Table 6 shows the mean efficacy of the butylene oxide adjuvant Sample 13 at several addition rates with the pesticide fomesafen compared to the adjuvant Brij 96V across four weed species, the mean values and a letter denoting which group each adjuvant belonged to are shown. As can be seen the butylene oxide adjuvant was more efficacious than the standard adjuvant across the weed species. In addition it was as effective at half the rate of the standard Brij96V (Sample 13 at 0.1% cf. Brij 96V at 0.2%). The level of addition of the butylene oxide adjuvant that was statistically better than no adjuvant was 0.025%. This is a very low level of adjuvant addition, indicating the remarkable efficacy of this adjuvant.

**Table 6**

| **Sample** | **Adjuvant rate (%)** | **Rank** | **Mean % kill** |
|---|---|---|---|
| Sample-13 | 0.5 | A | 61.805 |
| Sample 13 | 0.2 | B | 57.15 |
| Sample 13 | 0.1 | BC | 54.23 |
| Brij 96 | 0.2 | C | 53.33 |
| Sample 13 | 0.05 | D | 43.95 |
| Sample 13 | 0.025 | E | 38.12 |
| No adjuvant | 0 | F | 27.57 |

### EXAMPLE 6

In this example the butylene oxide adjuvant Sample 13 of Table 1 was compared to the adjuvant Tween™ 20. The herbicide mesotrione was applied at rates of 45 and 90 grams per hectare by a laboratory track sprayer to the weed species brachiaria platyphyla (BRAPL), digitaria sanguinalis (DIGSA), polygonum convolvulus (POLCO) and amaranthus tuberculatus (AMATU). In each case the experiments were replicated four times and the percentage damage to the weeds was assessed visually at time periods of 7, 14 and 21 days after application. The adjuvants was applied at a rate of 0.5% of the volume of the spray water.

The same statistical methodology that was applied in Example 3 was used here.

Table 7 shows the mean efficacy of the butylene oxide adjuvant Sample 13 at a rate of 0.5 % v/v with the pesticide mesotrione compared to the adjuvant Tween 20 across four weed species and compared to single weed species. The mean values and a letter denoting which group each adjuvant belonged to are shown. In addition the mean values split according to individual weed species are also shown. As can be seen the butylene oxide adjuvant was more efficacious than the standard adjuvant across the weed species, and was as good or better on individual weeds.

**Table 7**

| **Sample** | **All weeds** | **BRAPL** | **DIGSA** | **POLCO** | **AMATU** |
|---|---|---|---|---|---|
| Sample 13 | 69.986; A | 54.72; A | 56.94; A | 97.167; A | 71.11; A |
| Tween 20 | 63.861; B | 44.72; B | 48.33; B | 95.44; A | 66.94; AB |
| No adjuvant | 44.986; C | 20.27; C | 19.44; C | 77.16; B | 63.05; B |

### EXAMPLE 7

In this example the butylene oxide adjuvant Sample 13 of Table 1 was compared to the adjuvantBrij™96V. The herbicide mesotrione was applied to the weed species brachiaria platyphyla (BRAPL), digitaria sanguinalis (DIGSA), polygonum convolvulus (POLCO) and amaranthus tuberculatus (AMATU) at rates of 45 or 90 grams per hectare by a laboratory track sprayer. Each experiment was replicated four times and the percentage damage to the weeds was assessed visually at time periods of 7, 14 and 21 days after application. The adjuvants were applied at a rate of 0.2% of the volume of the spray water.

The same statistical methodology that was applied in Example 3 was used here.

Table 8 shows the mean efficacy of the butylene oxide adjuvant Sample 13 at a rate of 0.2% v/v with the pesticide mesotrione compared to the adjuvant Brij 96V across four weed species, and compared to single weed species. The mean values and a letter denoting which group each adjuvant belonged to are shown. In addition the mean values split according to individual weed species are also shown. As can be seen the butylene oxide adjuvant was more efficacious than the standard adjuvant across the weed species, and was as good or better on individual weeds.

**Table 8**

| **Sample** | **All weeds** | **BRAPL** | **DIGSA** | **POLCO** | **AMATU** |
|---|---|---|---|---|---|
| Sample 13 | 68.44; A | 51.66; A | 54.72; A | 97.11; A | 70.28; A |
| Brij 96V | 61.4; B | 41.94; B | 44.72; B | 93.66; B | 65.28; B |
| No adjuvant | 44.99; C | 20.27; C | 19.44; C | 77.16; C | 63.05; B |

### EXAMPLE 8

In this example the rate response of the butylene oxide adjuvant-Sample 13 of Table 1 was measured to display the excellent performance of this adjuvant at very low rates compared to the adjuvant Brij™96V. The herbicide mesotrione was applied to the weed species brachiaria platyphyla (BRAPL), digitaria sanguinalis (DIGSA), polygonum convolvulus (POLCO) and amaranthus tuberculatus (AMATU) at rates of 45 or 90 grams per hectare by a laboratory track sprayer. Each experiment was replicated four times and the percentage damage to the weeds was assessed visually at time periods of 7, 14 and 21 days after application. The butylene oxide adjuvant was applied at rates of 0.025, 0.05, 0.1, 0.2 and 0.5% w/v of the spray water. For comparison Brij96V was added at a rate of 0.2% w/v.

The same statistical methodology that was applied in Example 3 was used here.

Table 9 shows the mean efficacy of the butylene oxide adjuvant Sample 13 at several addition rates with the pesticide mesotrione compared to the adjuvant Brij 96V across four weed species. The mean values and a letter denoting which group each adjuvant belonged to are shown. As can be seen half the rate of the butylene oxide adjuvant was more efficacious than the standard adjuvant (0.1 % vs 0.2 %). In addition it was as effective at one quarter of the rate of the standard Brij96V (0.05 % vs 0.2%). The level of addition of the butylene oxide adjuvant that was statistically better than no adjuvant was 0.025 %. This is a very low level of adjuvant addition indicating the remarkable efficacy of this adjuvant.

**Table 9**

| **Sample** | **Adjuvant rate %** | **Rank** | **Mean % kill** | **Standard error** |
|---|---|---|---|---|
| Sample 13 | 0.5 | **A** | 70.0 | 1.8737 |
| Sample 13 | 0.2 | **AB** | 68.4 | |
| Sample 13 | 0.1 | **BC** | 66.2 | |
| Sample 13 | 0.05 | **CD** | 63.0 | |
| Brij 96 | 0.2 | **D** | 61.4 | |
| Sample 13 | 0.025 | **D** | 59.5 | |
| No adjuvant | 0 | **E** | 45.0 | |

### EXAMPLE 9

In this example the rate response of the butylene oxide adjuvant Sample 13 of Table 1 was measured to display the excellent performance of this adjuvant at very low rates. The herbicide pinoxaden was applied to the weed species lolium perenne (LOLPE), alopecurius myosuirides (ALOMY), setaria viridis (SETVI) and avena fatua (AVEFA) at rates of 7.5 or 15 grams per hectare by a laboratory track sprayer. Each experiment was replicated four times and the percentage damage to the weeds was assessed visually at time periods of 14 and 21 days after application. The butylene oxide adjuvant was applied at rates of 0.025, 0.05, 0.1, 0.2 and 0.5% w/v of the spray water. The same statistical methodology that was applied in Example 3 was used here.

Table 10 shows the mean efficacy of the butylene oxide adjuvant Sample 13 at several addition rates with the pesticide pinoxaden. Results are meaned across four weed species. The mean values and a letter denoting which group each adjuvant belonged to are shown. The results show that the butylene oxide adjuvant is efficacious at a very low level (0.025%) and that there is a strong rate response to the added adjuvant.

**Table 10**

| **Sample** | **Adjuvant rate %** | **Rank** | **Mean % kill** | **Standard error** |
|---|---|---|---|---|
| Sample 13 | 0.5 | A | 76.89 | 1.441 |
| Sample 13 | 0.2 | B | 73.89 | |
| Sample 13 | 0.1 | B | 73.45 | |
| Sample 13 | 0.05 | C | 67.53 | |
| Sample 13 | 0.025 | D | 60.76 | |
| No adjuvant | 0 | E | 4.72 | |

### EXAMPLE 10

In this example the butylene oxide adjuvant Sample 13 of Table 1 was compared to the commercial oil adjuvant blend Atplus™411F. The herbicide nicosulfuron was applied to the weed species chenopodium album (CHEAL), digitaria sanguinalis (DIGSA), setaria viridis (SETVI) and abutilon theophrasti (ABUTH) at rates of 30 or 60 grams per hectare by a laboratory track sprayer. Each experiment was replicated four times and the percentage damage to the weeds was assessed visually at time periods of 14 and 21 days after application. The adjuvants were applied at a rate of 0.5% of the volume of the spray water.

The same statistical methodology that was applied in Example 3 was used here. Table 11 shows the mean efficacy of the butylene oxide adjuvant Sample 13 at a rate of 0.5%v/v with the pesticide nicosulfuron compared to the oil adjuvant Atplus™411F across four weed species and compared to single weed species. The mean values and a letter denoting which group each adjuvant belonged to are shown. In addition the mean values split according to individual weed species are also shown. As can be seen the butylene oxide adjuvant was more efficacious than the standard adjuvant across the weed species, and was as good or better on individual weeds.

**Table 11**

| **Sample** | **All weeds** | **CHEAL** | **SETVI** | **DIGSA** | **ABUTH** |
|---|---|---|---|---|---|
| Sample 13 | 76.15; A | 84.58; A | 90; A | 81.25; A | 48.75; A |
| Atplus 411F | 66.75; B | 74.17; B | 81.58; B | 78.75; A | 32.5; B |
| No adjuvant | 20.94; C | 12.08; C | 41.67; C | 8.75; B | 21.25; C |

### EXAMPLE 11

In this example the butylene oxide adjuvant Sample 13 of Table 1 was compared to the commercial adjuvant tris 2-ethylhexyl phosphate (TEHP). The herbicide nicosulfuron was applied to the weed species chenopodium album (CHEAL), digitaria sanguinalis (DIGSA), setaria viridis (SETVI) and abutilon theophrasti (ABUTH)at rates of 30 or 60 grams per hectare by a laboratory track sprayer. Each experiment was replicated four times and the percentage damage to the weeds was assessed visually at time periods of 14 and 21 days after application. The adjuvants were applied at a rate of 0.2% of the volume of the spray water.

The same statistical methodology that was applied in Example 3 was used here.

Table 12 shows the mean efficacy of the butylene oxide adjuvant Sample 13 at a rate of 0.2% v/v with the pesticide nicosulfuron compared to the adjuvant TEHP the same rate across four weed species, and compared to single weed species. The mean values and a letter denoting which group each adjuvant belonged to are shown along with the standard error. In addition the mean values split according to individual weed species are also shown. As can be seen the butylene oxide adjuvant was more efficacious than the standard adjuvant across the weed species, and was as good or better on individual weeds.

**Table 12**

| **Sample** | **All weeds** | **CHEAL** | **SETVI** | **DIGSA** | **ABUTH** |
|---|---|---|---|---|---|
| Sample 13 | 68.23; A | 76.67; A | 80; A | 73.33; A | 42.92; A |
| TEHP | 58.12; B | 68.33; B | 77.08; A | 69.17; A | 17.92; B |
| No adjuvant | 20.94; C | 12.08; C | 41.67; B | 8.75; B | 21.25; B |

### EXAMPLE 12

In this example the rate response of the butylene oxide adjuvant Sample 13 of Table 1 was measured and compared to the adjuvant TEHP. The herbicide nicosufuron was applied to the weed species chenopodium album (CHEAL), digitaria sanguinalis (DIGSA), setaria viridis (SETVI) and abutilon theophrasti (ABUTH) at rates of 30 to 60 grams per hectare by a laboratory track sprayer. Each experiment was replicated four times and the percentage damage to the weeds was assessed visually at time periods of 14 and 21 days after application. The butylene oxide adjuvant was applied at rates of 0.025, 0.05, 0.1, 0.2 and 0.5% w/v of the spray water. For comparison TEHP was added at a rate of 0.2% w/v.

The same statistical methodology that was applied in Example 3 was used here.

Table 13 shows the mean efficacy of the butylene oxide adjuvant Sample 13 at several addition rates with the pesticide nicosulfuron. Results are meaned across four weed species. As a comparison the adjuvant TEHP was added at 0.2% v/v. The mean values and a letter denoting which group each adjuvant belonged to are shown. As can be seen half the rate of the butylene oxide adjuvant was more efficacious than the standard adjuvant (Sample 13 at 0.1% compared to TEHP at 0.2%). In addition it was as effective at one quarter of the rate of the standard TEHP (0.05% compared to 0.2%). The level of addition of the butylene oxide adjuvant that was statistically better than no adjuvant was 0.025%. This is a very low level of adjuvant addition, indicating the remarkable efficacy of this adjuvant.

**Table 13**

| **Sample** | **Adjuvant rate %** | **Rank** | **Mean % kill** | **Standard error** |
|---|---|---|---|---|
| Sample 13 | 0.5 | A | 76.14 | 1.803 |
| Sample 13 | 0.2 | B | 68.23 | |
| Sample 13 | 0.1 | C | 63.23 | |
| TEHP | 0.2 | D | 58.12 | |
| Sample 13 | 0.05 | D | 56.35 | |
| Sample 13 | 0.025 | E | 51.87 | |
| No adjuvant | 0 | F | 20.93 | |

### EXAMPLE 13

In this example the responses of the butylene oxide adjuvants 13, 17, 19, 21, 22 and 23of Table 1 were measured at an application rate of 0.2% of the volume of the spray solution used. They were compared to the commercial tank mix adjuvant Atplus™411F, which was applied at the recommended rate of 0.5% by volume. The herbicide nicosufuron was applied to the weed species chenopodium album (CHEAL), digitaria sanguinalis (DIGSA), setaria viridis (SETVI) and abutilon theophrasti (ABUTH) at rates of 30 or 60 grams per hectare by a laboratory track sprayer. Each experiment was replicated four times and the percentage damage to the weeds was assessed visually at time periods of 14 and 21 days after application.

The same statistical methodology that was applied in Example 3 was used here.

Table 14 shows the mean efficacy of six butylene oxide adjuvants at an addition rate of 0.2% with the pesticide nicosulfuron. Results are meaned across two pesticide rates and each sample was replicated four times. As a comparison the adjuvant Atplus™ 411F was added at 0.5% v/v. The mean values and a letter denoting which group each adjuvant belonged to are shown. The data shows that the butylene oxide adjuvants were at least as good as the standard which was used at a higher rate than the butylene oxide adjuvants (0.2% compared to 0.5%).

**Table 14**

| **Sample** | **All weeds** | **ABUTH** | **CHEAL** | **DIGSA** | **SETVI** |
|---|---|---|---|---|---|
| Sample 13 | 78.44; A | 65.42; A | 85; A | 67.5; A | 95.83; A |
| Sample 21 | 76.45; AB | 66.25; A | 85; A | 60; AB | 94.58; AB |
| Sample 22 | 76.35; AB | 61.67; A | 85.83; A | 62.5; AB | 95.42; A |
| Sample 19 | 75.81; AB | 65; A | 84.17; A | 58.33; BC | 95.75; A |
| Sample 23 | 74.16; AB | 62.92; A | 82.08; A | 57.92; BC | 93.75; AB |
| Atplus 411 F | 70.73; B | 60.83; A | 74.17; B | 56.67; BC | 91.25; B |
| Sample 17 | 69.37; B | 58.75; A | 76.67; B | 50.83; C | 91.25; B |
| No adjuvant | 37.5; C | 25.83; B | 24.16; C | 18.33; D | 81.67; C |

### EXAMPLE 14

In this example the responses of the adjuvants 13, 17, 19, 21, 22 and 23 were measured at an application rate of 0.2% of the volume of the spray solution used. They were compared to the adjuvant tris 2-ethylhexyl phosphate (TEHP), which was applied at the rate of 0.5% by volume. The herbicide pinoxaden was applied at rates of 7.5 or 15 grams per hectare by a laboratory track sprayer to the weed species lolium perenne (LOLPE), alopecurius myosuirides (ALOMY), setaria viridis (SETVI) and avena fatua (AVEFA). In each case the experiments were replicated four times and the percentage damage to the weeds was assessed visually at time periods of 14 and 21 days after application.

The same statistical methodology that was applied in Example 3 was used here.

Table 15 shows the mean efficacy of six butylene oxide adjuvants at an addition rate of 0.2% with the pesticide pinoxaden. Results are meaned across two pesticide rates and each sample was replicated four times. As a comparison the adjuvant tris 2 ethylhexyl phosphate was added at 0.5% v/v. The mean values and a letter denoting which group each adjuvant belonged to are shown along with the standard error. The standard TEHP was used at a higher rate than the butylene oxide adjuvants (0.2% compared to 0.5%).

**Table 15**

| **Sample** | **All weeds** | **SETVI** | **LOLPE** | **AVEFA** | **ALOMY** |
|---|---|---|---|---|---|
| Sample 23 | 76.98; A | 78.75; ABC | 78.33; AB | 78.33; AB | 72.5; A |
| Sample 13 | 76.98; A | 83.33; A | 77.5; AB | 80.42; A | 66.67; B |
| Sample 19 | 76.25; A | 80; AB | 77.08; AB | 77.08; AB | 70.83; AB |
| TEHP | 75.63; A | 77.5; ABC | 82.5; A | 73.75; AB | 68.75; AB |
| Sample 22 | 73.96; AB | 78.33; ABC | 72.5; BC | 75.83; AB | 69.17; AB |
| Sample 21 | 72.19; AB | 71.25; C | 76.67; AB | 71.66; B | 69.17; AB |
| Sample 17 | 68.02; B | 72.92; BC | 68.75; C | 73.33; AB | 57.08; C |
| No adjuvant | 15.62; C | 17.08; D | 7.08; D | 23.33; C | 15; D |

### EXAMPLE 15

In this example the rate response of the butylene oxide sample 27 of Table 1 was measured, displaying the excellent performance of this adjuvant compared to the commercial adjuvant Turbocharge™, used at a rate of 0.5% by volume. The herbicide fomesafen was applied to the weed species xanthium strumarium (XANST), setaria viridis (SETVI), abutilon theophrasti (ABUTH), and chenopodium album (CHEAL)at rates of 60 or 120 grams per hectare by a laboratory track sprayer. Each experiment was replicated four times and the percentage damage to the weeds was assessed visually at time periods of 7, 14 and 21 days after application. Sample 27 was applied at a rate of 0.2% of the volume of the spray water.

The same statistical methodology that was applied in Example 3 was used here Table 16 shows the mean efficacy of the butylene oxide adjuvant 27 at a rate of 0.2% v/v with the pesticide fomesafen compared to the adjuvant Turbocharge at a rate of 0.5%. The mean values and a letter denoting which group each adjuvant belonged to are shown along with the standard error. As can be seen the butylene oxide adjuvant was as effective as Turbocharge even although it was applied at 0.2% as opposed to 0.5% for the commercial adjuvant.

**Table 16**

| **Sample** | **All weeds** | **XANST** | **SETVI** | **CHEAL** | **ABUTH** |
|---|---|---|---|---|---|
| Sample 27 | 51.01; A | 73.44; A | 33.44; A | 74.3; A | 22.86; B |
| Turbocharge | 52.89; A | 72.33; A | 28.72; B | 75.69; A | 34.81; A |
| No adjuvant | 22.94; B | 31.55; B | 13.78; C | 30.69; B | 15.75; C |

### EXAMPLE 16

In this example the rate response of the butylene oxide adjuvant 27 of Table 1 was measured to display the excellent performance of this adjuvant compared to the commercial adjuvant Tween™20, used at a rate of 0.5% by volume. The herbicide mesotrione was applied to the weed species brachiaria platyphyla (BRAPL), digitaria sanguinalis (DIGSA), polygonum convolvulus (POLCO) and amaranthus tuberculatus (AMATU) at rates of 45 or 90 grams per hectare by a laboratory track sprayer. Each experiment was replicated four times and the percentage damage to the weeds was assessed visually at time periods of 7, 14 and 21 days after application. Sample 27 was applied at a rate of 0.2% of the volume of the spray water.

The same statistical methodology that was applied in Example 3 was used here.

Table 17 shows the mean efficacy of the butylene oxide adjuvant 27 at a rate of 0.2% v/v with the pesticide mesotrione compared to the adjuvant Tween 20 at a rate of 0.5%. The mean values and a letter denoting which group each adjuvant belonged to are shown. As can be seen the butylene oxide adjuvant was as effective as Tween 20 even although it was applied at 0.2% as opposed to 0.5% for the commercial adjuvant.

**Table 17**

| **Sample** | **All weeds** | **POLCO** | **DIGSA** | **BRAPL** | **AMATU** |
|---|---|---|---|---|---|
| Sample 27 | 57.26; A | 75.44; A | 44.99; A | 36.94; A | 71.64; A |
| Tween 20 | 54.54; A | 73.61; A | 41.67; A | 33.06; B | 69.86; A |
| No adjuvant | 34.84; B | 55.5; B | 20.55; B | 19.17; C | 44.13; B |

### EXAMPLE 17

In this example the response of butylene oxide sample 27 of Table 1 was measured at an application rate of 0.2% of the volume of the spray solution used. It was compared to the adjuvant Genopol™X080, which was also applied at a rate of 0.2% by volume. This adjuvant has the same alkyl chain and ethylene oxide head group as Sample 27 however it does not contain the butylene oxide moiety. The herbicide pinoxaden was applied at rates of 7.5 or 15 grams per hectare by a laboratory track sprayer to the weed species lolium perenne (LOLPE), alopecurius myosuirides (ALOMY), setaria viridis (SETVI) and avena fatua (AVEFA). Each experiment was replicated four times and the percentage damage to the weeds was assessed visually at time periods of 14 and 21 days after application.

The same statistical methodology that was applied in Example 3 was used here.

Table 18 shows the mean efficacy of the butylene oxide adjuvant 27 at a rate of 0.2% v/v with the pesticide pinoxaden compared to the adjuvant Genapol X080 at the same rate. The mean values and a letter denoting which group each adjuvant belonged to are shown. The data shows that the butylene oxide adjuvants was significantly more efficacious than Genapol X080 across the range of weed species tested.

**Table 18**

| **Sample** | **All weeds** | **SETVI** | **LOLPE** | **AVEFA** | **ALOMY** |
|---|---|---|---|---|---|
| Sample 27 | 71.06; A | 84.4; A | 59.22; A | 82.3; A | 58.31; A |
| Genopol X080 | 52.08; B | 70.67; B | 20.97; B | 75.47; B | 41.23; B |
| No adjuvant | 7.44; C | 4.75; C | 6.53; C | 5.74; C | 12.73; C |

### EXAMPLE 18

In this example the response of butylene oxide sample 27 of Table 1 was measured at an application rate of 0.2% by volume of the spray solution used. It was compared to the commercial tank mix adjuvant Atplus™41 IF, which was applied at the recommended rate of 0.5% by volume, and to the adjuvant Genapol™X080 which was applied at 0.2%. This adjuvant has the same alkyl chain and ethylene oxide head group as sample 27 however it does not contain the butylene oxide moiety. The herbicide nicosufuron was applied at rates of 30 or 60 grams per hectare by a laboratory track sprayer to the weed species chenopodium album (CHEAL), digitaria sanguinalis (DIGSA), setaria viridis (SETVI) and abutilon theophrasti (ABUTH). In each case the experiments were replicated four times and the percentage damage to the weeds was assessed visually at time periods of 14 and 21 days after application.

The same statistical methodology that was applied in Example 3 was used here.

Table 19 shows the mean efficacy of the butylene oxide adjuvant 27 at a rate of 0.2 % v/v with the pesticide nicosulfuron compared to the adjuvant Genapol X080 at the same rate, and to Atplus 411F applied at the higher rate of 0.5%. The mean values and a letter denoting which group each adjuvant belonged to are shown. The data shows that the butylene oxide adjuvants was as good as Atplus 411F which was used at a higher rate than the butylene oxide adjuvants (0.2% compared to 0.5%). It was more efficacious than Genapol X080.

**Table 19**

| **Sample** | **All weeds** | **SETVI** | **DIGSA** | **CHEAL** | **ABUTH** |
|---|---|---|---|---|---|
| Sample 27 | 70.26; A | 45.13; A | 79.55; A | 84.58; A | 71.76; A |
| Atplus 411F | 65.74; A | 25.96; B | 76.05; A | 85.01; A | 75.93; A |
| Genopol X080 | 46.47; B | 19.72; C | 42.71; B | 70.42; B | 53.01; B |
| No adjuvant | 21.39; C | 14.17; D | 22.59; C | 15.2; C | 33.6; C |

### EXAMPLE 19

In this example the rate response of the butylene oxide adjuvant 13 of Table 1 was measured to display the excellent performance of this adjuvant compared to the commercial adjuvant Turbocharge™, used at a rate of 0.5% by volume. The herbicide fomesafen was applied at rates of 60, 90 or 120 grams per hectare by a laboratory track sprayer to the weed species xanthium strumarium (XANST), abutilon theophrasti (ABUTH), and chenopodium album (CHEAL). In each case the experiments were replicated four times and the percentage damage to the weeds was assessed visually at time periods of 14 and 21 days after application. Sample 13 was applied at a rate of 0.2% of the volume of the spray water.

The same statistical methodology that was applied in Example 3 was used here.

Table 21 shows the mean efficacy of the butylene oxide adjuvant 13 at a rate of 0.2% v/v with the pesticide fomesafen compared to the adjuvant Turbocharge applied at the higher rate of 0.5%. The mean values and a letter denoting which group each adjuvant belonged to are shown. As can be seen the butylene oxide adjuvant was more effective than Turbocharge even although it was applied at 0.2% as opposed to 0.5% for the commercial adjuvant.

**Table 20**

| **Sample** | **All weeds** | **XANST** | **CHEAL** | **ABUTH** |
|---|---|---|---|---|
| Sample 13 | 51.24; A | 92.56; A | 62.5; A | 41.39; A |
| Turbocharge | 39.43; B | 77.44; B | 40; B | 35; B |
| No adjuvant | 23.61; C | 45; C | 24.44; C | 25; C |

### EXAMPLE 20

In this example the rate response of the butylene oxide adjuvant 13 of Table 1 was measured to display the excellent performance of this adjuvant compared to the commercial adjuvant Tween™20, used at a rate of 0.5% by volume. The herbicide mesotrione was applied at rates of 30, 60 or 90 grams per hectare by a laboratory track sprayer to the weed species brachiaria platyphyla (BRAPL), digitaria sanguinalis (DIGSA), and polygonum convolvulus (POLCO). Sample 13 was applied at a rate of 0.2% of the volume of the spray water. In each case the experiments were replicated four times and the percentage damage to the weeds was assessed visually at time periods of 14 and 21 days after application.

The same statistical methodology that was applied in Example 3 was used here.

Table 21 shows the mean efficacy of the butylene oxide adjuvant 13 at a rate of 0.2% v/v with the pesticide mesotrione compared to the adjuvant Tween 20 applied at the higher rate of 0.5%. The mean values and a letter denoting which group each adjuvant belonged to are shown. As can be seen the butylene oxide adjuvant was more effective than Tween 20 even though it was applied at 0.2% as opposed to 0.5 % for the commercial adjuvant.

**Table 21**

| **Sample** | **All weeds** | **POLCO** | **DIGSA** | **BRAPL** |
|---|---|---|---|---|
| Sample 13 | 28.89; A | 36.11; A | 47.5; A | 39.14; A |
| Tween 20 | 26.11; B | 32.77; B | 45.55; A | 26.11; B |
| No adjuvant | 13.61; C | 27.78; C | 17.5; B | 9.17; C |

### EXAMPLE 21

In this example the response of the butylene oxide adjuvant 13 of Table 1 was measured at an application rate of 0.2% of the volume of the spray solution used. It was compared to the commercial tank mix adjuvant Atplus™411F, which was applied at the recommended rate of 0.5% by volume. The herbicide nicosufuron was applied at rates of 30, 45 or 60 grams per hectare by a laboratory track sprayer to the weed species chenopodium album (CHEAL), digitaria sanguinalis (DIGSA), and abutilon theophrasti (ABUTH). In each case the experiments were replicated four times and the percentage damage to the weeds was assessed visually at time periods of 14 and 21 days after application.

The same statistical methodology that was applied in Example 3 was used here.

Table 22 shows the mean efficacy of the butylene oxide adjuvant 13 at a rate of 0.2% v/v with the pesticide nicosulfuron compared to the adjuvant Atplus 411F applied at the higher rate of 0.5%. The mean values and a letter denoting which group each adjuvant belonged to are shown. The data shows that the butylene oxide adjuvant was as good as Atplus 411F which was used at a higher rate than the butylene oxide adjuvant (0.5% compared to 0.2%).

**Table 22**

| **Sample** | **All weeds** | **DIGSA** | **CHEAL** | **ABUTH** |
|---|---|---|---|---|
| Sample 13 | 67.31; A | 78.61; A | 88.33; A | 35; A |
| Atplus 411F | 63.61; A | 75.55; B | 88.06; A | 27.22; B |
| No adjuvant | 8.11; B | 4.05; C | 0.56; B | 19.72; C |

### EXAMPLE 22

In this example the response of the butylene oxide adjuvant 13 of Table 1 was measured at an application rate of 0.2% of the volume of the spray solution used. It was compared to the adjuvant tris 2-ethylhexyl phosphate [TEHP], which was applied at the higher rate of 0.5% by volume. The herbicide pinoxaden was applied at rates of 7.5, 11.25 or 15 grams per hectare by a laboratory track sprayer to the weed species lolium perenne (LQLPE), alopecurius myosuirides (ALOMY), and avena fatua (AVEFA). In each case the experiments were replicated four times and the percentage damage to the weeds was assessed visually at time periods of 14 and 21 days after application.

The same statistical methodology that was applied in Example 3 was used here.

Table 23 shows the mean efficacy of butylene oxide adjuvant 13 at a rate of 0.2% v/v with the pesticide pinoxaden compared to the adjuvant tris 2-ethylhexyl phosphate applied at the higher rate of 0.5%. The mean values and a letter denoting which group each adjuvant belonged to are shown. The data shows that the butylene oxide adjuvant was as effective as TEHP across the range of weed species tested.

**Table 23**

| **Sample** | **All weeds** | **LOLPE** | **AVEFA** | **ALOMY** |
|---|---|---|---|---|
| Sample 13 | 58.05; A | 53.89; B | 76.94; A | 43.33; A |
| TEHP | 59.91; A | 58.06; A | 78.33; A | 43.33; A |
| No adjuvant | 3.43; B | 2.22; C | 6.39; B | 1.67; B |

### EXAMPLE 23

In this example the rate response of 22 butylene oxide adjuvants of Table 1 were measured at an adjuvant rate of 0.2% by volume, displaying excellent performance of this adjuvant compared to the commercial adjuvant Turbocharge, which was used at a rate of 0.5% by volume. The herbicide mesotrione was applied at rates of 60 or 120 grams per hectare by a laboratory track sprayer to the weed species brachiaria platyphyla (BRAPP), digitaria sanguinalis (DIGSA), abutilon theophrasti (ABUTH) and amaranthus retroflexus (AMARE). In each case the experiments were replicated four times and the percentage damage to the weeds was assessed visually at time periods of 14 and 21 days after application. All the butylene oxide adjuvants were applied at a rate of 0.2% volume of the spray water whereas Turbocharge was used at 0.5%.

The same statistical methodology that was applied in Example 3 was used here.

Table 23 shows the mean efficacy of 22 butylene oxide adjuvants used at a rate of 0.2% v/v with the pesticide mesotrione compared to the adjuvant Turbocharge applied at the higher rate of 0.5%. The mean values and a letter denoting which group each adjuvant belonged to are shown. As can be seen most of the butylene oxide adjuvants were more effective than Turbocharge even although they were applied at 0.2% as opposed to 0.5% for the commercial adjuvant. All of the butylene adjuvants were as good as Turbocharge.

**Table 23**

| **Sample** | **Group** | **Mean kill across weeds %** |
|---|---|---|
| 3 | A | 84.69 |
| 27 | AB | 84.27 |
| 26 | ABC | 83.85 |
| 21 | ABCD | 83.65 |
| 13 | ABCDE | 83.44 |
| 20 | ABCDE | 83.44 |
| 23 | ABCDE | 83.44 |
| 19 | ABCDEF | 83.13 |
| 7 | ABCDEF | 83.02 |
| 2 | ABCDEF | 82.60 |
| 6 | ABCDEF | 82.60 |
| 1 | BCDEFG | 81.98 |
| 22 | CDEFGH | 81.88 |
| 18 | CDEFGH | 81.77 |
| 25 | CDEFGH | 81.67 |
| 10 | DEFGHI | 81.35 |
| 17 | EFGHI | 81.25 |
| 11 | FGHI | 80.94 |
| 8 | GHIJ | 80.21 |
| 24 | HIJ | 79.58 |
| 16 | IJ | 79.27 |
| Turbocharge | J | 78.44 |
| 5 | J | 78.13 |
| No adjuvant | K | 69.48 |

### EXAMPLE 24

In this example the responses of the butylene oxide adjuvants 7, 14, 24 and 25 of -Table1 were measured at an application rate of 0.2% of the volume of the spray solution used. They were compared to the adjuvant Brij™96V, which was applied at the same rate. The herbicide pinoxaden was applied to the weed species lolium perenne (LOLPE), alopecurius myosuirides (ALOMY), setaria viridis (SETVI) and avena fatua (AVEFA) at rates of 7.5 or 15 grams per hectare by a laboratory track sprayer. Each experiment was replicated three times and the percentage damage to the weeds was assessed visually 13 days after application.

The same statistical methodology that was applied in Example 3 was used here.

Table 25 shows the mean efficacy of 4 butylene oxide adjuvants used at a rate of 0.2% v/v with the pesticide pinoxaden compared to the adjuvant Brij 96V applied at the same rate. The mean values and a letter denoting which group each adjuvant belonged to are shown. The data shows that two of the butylene oxide adjuvants were more effective than Brij 96V and two were as good as Brij 96V across the range of weed species tested.

**Table 25**

| **Sample** | **Group** | **Mean kill % all weeds** |
|---|---|---|
| 7 | A | 72.3 |
| 14 | A | 67.3 |
| 25 | B | 57.3 |
| Brij 96V | B | 55.0 |
| 24 | B | 52.9 |
| No adjuvant | C | 16.3 |

### EXAMPLE 25

In this example the rate responses of four butylene oxide adjuvants of Table 1, applied at 0.2%, displayed excellent performance when compared to the commercial adjuvant Turbocharge™, used at a rate of 0.5%; and to tris 2-ethylhexyl phosphate (TEHP) also used at 0.5%. The herbicide fomesafen was applied to the weed species xanthium strumarium (XANST), abutilon theophrasti (ABUTH), setaria viridis (SETVI) and chenopodium album (CHEAL) at rates of 60 or 120 grams per hectare by a laboratory track sprayer. Each experiment was replicated six times and the percentage damage to the weeds was assessed visually at time periods of 7 and 13 days after application.

The same statistical methodology that was applied in Example 3 was used here.

Table 26 shows the mean efficacy of 4 butylene oxide adjuvants used at a rate of 0.2 v/v with the pesticide fomesafen compared to the adjuvants tris 2-ethylhexyl phosphate (TEHP) and Turbocharge. The latter two adjuvants were applied at the higher rate of 0.5% v/v. The mean values and a letter denoting which group each adjuvant belonged to are shown along with the standard error. As can be seen the performance of all four of the butylene oxide adjuvants was at least as good as the standard Turbocharge and most of the adjuvants were as good as or better than TEHP.

**Table 26**

| **Sample** | **Group** | **Mean kill across weeds (%)** |
|---|---|---|
| 24 | A | 78.2 |
| 7 | B | 73.6 |
| 25 | B | 73.1 |
| TEHP | B | 72.7 |
| Turbocharge | C | 68.7 |
| 14 | C | 66.1 |
| None | D | 47.0 |

### Example (according to the invention) 26

In this example the rate response of sample 28 of Table 1, a non-ethoxylated adjuvant, applied at 0.2% by volume was measured to display the performance of the adjuvant compared to the commercial adjuvant Turbocharge, used at a rate of 0.5%. The herbicide fomesafen was applied to the weed species xanthium strumarium (XANST), abutilon theophrasti (ABUTH), setaria viridis (SETVI) and chenopodium album (CHEAL) at rates of 60 or 120 grams per hectare by a laboratory track sprayer. Each experiment was replicated three times and the percentage damage to the weeds was assessed visually at time periods of 14 and 21 days after application.

The same statistical methodology that was applied in Example 3 was used here. Table 27 shows the mean efficacy of a non-ethoxylated butylene oxide adjuvant used at a rate of 0.2% v/v with the pesticide fomesafen compared to the adjuvant Turbocharge. Turbocharge was applied at the higher rate of 0.5%. The mean values and a letter denoting which group each adjuvant belonged to are shown. As can be seen the performance of the butylene oxide adjuvant was as good as the standard Turbocharge.

**Table 27**

| **Sample** | **Group** | **Mean kill (%)** |
|---|---|---|
| Turbocharge | A | 67.6 |
| Sample 28 | A | 64.7 |
| No adjuvant | B | 37.7 |

### Example (according to the invention) 27

In this example the responses of sample 28 of table 1, a non-ethoxylated butylene oxide adjuvant was compared to tris 2-ethylhexyl phosphate (TEHP). The butylene oxide adjuvant was applied at an application rate of 0.2% by volume of the spray solution used whereas TEHP was applied at 0.5%. The herbicide pinoxaden was applied to the weed species lolium perenne (LOLPE), alopecurius myosuirides (ALOMY), setaria viridis (SETVI) and avena fatua (AVEFA) at rates of 7.5 or 15 grams per hectare by a laboratory track sprayer. Each experiment was replicated three times and the percentage damage to the weeds was assessed visually 14 and 21 days after application.

The same statistical methodology that was applied in Example 3 was used here.

Table 28 shows the mean efficacy of a non-ethoxylated butylene oxide adjuvant compared to TEHP with the pesticide pinoxaden. The rate of the former was 0.2% whereas the latter was applied at 0.5%. The mean values and a letter denoting which group each adjuvant belonged to are shown. The data shows that the butylene oxide adjuvant was as effective as TEHP across the range of weed species tested.

**Table 28**

| **Sample** | **Group** | **Mean kill (%)** |
|---|---|---|
| TEHP | A | 92.6 |
| Sample 28 | A | 91.0 |
| No adjuvant | B | 17.4 |

### Example (according to the invention) 28

In this example the rate response of a non-ethoxylated butylene oxide adjuvant was measured at an adjuvant rate of 0.2% by volume in order to display the excellent performance of this adjuvant compared to the commercial adjuvant' Tween 20, which was used at a rate of 0.5% by volume. The herbicide mesotrione was applied at rates of 45 or 90 grams per hectare by a laboratory track sprayer to the weed species brachiaria decumbens (BRADE), digitaria sanguinalis (DIGSA), polygonum convolvulus (POLCO) and amaranthus retroflexus (AMARE). In each case the experiments were replicated three times and the percentage damage to the weeds was assessed visually at time periods of 7, 14 and 21 days after application. The butylene oxide adjuvant was applied at a rate of 0.2% of the volume of the spray water whereas Tween 20 was used at 0.5%.

The same statistical methodology that was applied in Example 3 was used here.

Table 29 shows the mean efficacy of an unethoxylated butylene oxide adjuvant compared to Tween 20 with the pesticide mesotrione. The rate of the former was 0.2% whereas the latter was applied at 0.5%. The mean values and a letter denoting which group each adjuvant belonged to are shown. As can be seen the butylene oxide adjuvant was as effective as Tween 20 even though it was applied at 0.2% as opposed to 0.5% for the commercial adjuvant.

**Table 29**

| **Sample** | **Group** | **Mean kill %** |
|---|---|---|
| Sample 28 | A | 68.8 |
| Tween 20 | A | 65.8 |
| No adjuvant | B | 47.2 |

### Example (according to the invention) 29

In this example the response of sample 28 of Table 1, a non-ethoxylated butylene oxide adjuvant was measured at an application rate of 0.2% of the volume of the spray solution used. It was compared to the commercial tank mix adjuvant Atplus™411F, which was applied at the recommended rate of 0.5% by volume. The herbicide nicosufuron was applied at rates of 30 or 60 grams per hectare by a laboratory track sprayer to the weed species chenopodium album (CHEAL), digitaria sanguinalis (DIGSA), setaria viridis (SETVI) and abutilon theophrasti (ABUTH). In each case the experiments were replicated three times and the percentage damage to the weeds was assessed visually at time periods of 14 and 21 days after application.

The same statistical methodology that was applied in Example 3 was used here.

Table 30 shows the mean efficacy of a non-ethoxylated butylene oxide adjuvant compared to Atplus 411F with the pesticide nicosulfuron. The rate of the former was 0.2% whereas the latter was applied at 0.5%. The mean values and a letter denoting which group each adjuvant belonged to are shown along with the standard error. The data shows that the butylene oxide adjuvant was as good as Atplus 411F which was used at a higher rate than the butylene oxide adjuvants (0.2% compared to 0.5%).

**Table 30**

| **Sample** | **Group** | **Mean kill %** |
|---|---|---|
| ATPLUS 411 F | A | 90.4 |
| Sample 28 | A | 89.2 |
| No adjuvant | B | 75.0 |

### Example (according to the invention) 30

This example shows that samples 29 and 30 from Table 1 behave as adjuvants for fungicides used against the fungus septoria tritici. Wheat was sprayed with water at a rate of 200 litres per hectare, the water containing either isopyrazam or epoxyconazole, at a concentration which enabled a pesticide application rate of 0.6, 2, 6 or 20 grams per hectare. Sample 29 was added at a rate of 0.2% v/v of the spray volume used and sample 30 was added at a rate of 0.1 % v/v. As a comparison to these adjuvants the same formulation was tested without the adjuvant, as a standard. Each experiment was replicated 12 times and the results were averaged at each rate. Plants were examined for curative effects. The percentage disease on each sample was assessed visually and averaged across the replicates at each rate. This was converted to a percentage control by comparison to the disease level on plants which were sprayed using a blank spray application where the pesticide was not included. Table 31 shows the percentage septoria control for the two adjuvants used with the four levels of isopyrazam as well as the blank formulation. Table 32 shows the percentage septoria control for the two adjuvants used with the four levels of epoxyconazole as well as the blank formulation. In each case it can be seen that the adjuvants have improved the performance of the fungicide.

**Table 31**

| **Isopyrazam g/ha** | **Control** | **Sample 30** | **Sample 31** |
|---|---|---|---|
| 20 | **21** | **100** | **99** |
| 6 | **35** | **85** | **85** |
| 2 | **21** | **53** | **26** |
| 0.6 | **18** | **12** | **0** |

**Table 32**

| **Epoxyconazole g/ha** | **Control** | **Sample 30** | **Sample 31** |
|---|---|---|---|
| 20 | **29** | **99** | **100** |
| 6 | **11** | **99** | **99** |
| 2 | **22** | **88** | **90** |
| 0.6 | **4** | **45** | **54** |

### Example (according to the invention) 31

This is an insecticide example: it shows that sample 28 from Table 1 behaves as an adjuvant for the insecticide thiamethoxam against *Aphis craccivora.* The lower sides of French bean leaves were infested with an aphid population *Aphis craccivora* of mixed ages contained in clip cages. The upper sides of the leaves were sprayed with the test solutions, 1 day after aphid infestation. French bean was sprayed with water at a rate of 200 litres per hectare, the water containing 3, 6, 12.5 and 25 ppm thiamethoxam. Sample 28 was added at a rate of 0.1% v/v of the spray volume used. As a comparison to this adjuvant the same formulation was tested without the adjuvant, as a standard. 5 days after spray application, the aphids were checked visually for mortality. Each experiment was replicated twice and the results were averaged at each rate. In the control experiment the beans were sprayed with water and no mortality was observed.

**Table 33**

| Treatment | 3ppm thiamethoxam % mortality | 6ppm thiamethoxam % mortality | 12.5 ppm thiamethoxam % mortality | 25 ppm thiamethoxam % mortality |
|---|---|---|---|---|
| Actara WG25 | 0 | 70 | 99 | 100 |
| Actara WG25 + 0.1% v/v sample 28 | 50 | 90 | 97.5 | 100 |

| | | | | |
|---|---|---|---|---|
| Actara™ WG25 is a commercial product containing thiamethoxam | | | | |

### Example (according to the invention) 32

This is a Phytotoxicity example: it shows that sample 28 from Table 1 is not phytotoxic to soybean, French bean and Chinese cabbage. The plants were sprayed with water at a rate of approximately 500 litres per hectare, the water containing 0.1% v/v or 0.2% v/v adjuvant. The plants were assessed for phytotoxicity 7 days after spray application. Each experiment was replicated twice and the results averaged. In the control experiment the plants were sprayed with water and no phytotoxicity was observed. The results show that the adjuvant sample 28 is safer to the crops than the alcohol ethoxylate adjuvant Genapol O100.

**Table 34**

| | Soybean % phytotoxicity | French bean % phytotoxicity | Chinese cabbage % phytotoxicity |
|---|---|---|---|
| 0.1%v/v Sample 28 | 0 | 0 | 0 |
| 0.2%v/v Sample 28 | 1 | 0 | 0 |
| 0.1%v/v Genapol O100 | 3.5 | 5 | 2 |
| 0.2%v/v Genapol O100 | 15 | 10 | 10 |

| | | | |
|---|---|---|---|
| Genapol™ O100 is a commercial surfactant from Clariant, an oleyl ethoxylate with 10 moles ethylene oxide. | | | |

## Claims

1. A compound of formula (I)
R₁O[BO]₄H (I)
where BO is butylene oxide; and R₁ is C₁₂₋₁₅ alkyl or oleyl.

## Patentansprüche

1. Verbindung der Formel (I)
R₁O[BO]₄H (I)
wobei BO für Butylenoxid steht und R₁ für C₁₂₋₁₅-Alkyl oder Oleyl steht.

## Revendications

1. Composé de formule (I)
R₁O[BO]₄H (I)
dans laquelle BO est oxyde de butylène ; et R₁ est C₁₂₋₁₅ alkyle ou oléyle.
